# EUROPEAN PATENT APPLICATION

(11) **EP 2 554 158 A1**
(43) Date of publication of application: **06.02.2013**
(21) Application number: 11762392.6
(22) Date of filing: 18.02.2011
(51) Int. Cl.: A61K 8/86, A61K 8/81, A61Q 5/06

(54) **SOLID OR POWDERY HAIR STYLING COMPOSITION**

(30) Priority: 31.03.2010 JP 2010081408
(71) Applicant: Shiseido Company, Ltd., Chuo-ku Tokyo 104-8010 (JP)
(72) Inventor: HAGIHARA Motohumi, Yokohama-shi Kanagawa 224-8558 (JP); KUROKAWA Kenji, Yokohama-shi Kanagawa 224-8558 (JP); HAYASHIGE Tomonari, Tokyo 105-0021 (JP); KATAYAMA Mika, Yokohama-shi Kanagawa 224-8558 (JP); TOYODA Tomonori, Yokohama-shi Kanagawa 224-8558 (JP)
(74) Representative: TER MEER - STEINMEISTER & PARTNER GbR
(86) International application number: PCT/JP2011/053443
(87) International publication number: WO 2011/122151

(57) **Abstract**

The present invention provides a solid or powder hairdressing composition comprising the following (a) ingredient and (b) ingredient:
(a) hair styling agent, and
(b) polyethylene glycol having a mass-average molecular weight of 1,000-25,000.

The object of the present invention is to provide a hairdressing that is used on towel-dried wet hair after hair washing, not a conventional liquid, paste, or cream hairdressing but a solid or powder hairdressing that contains none or a drastically reduced amount of ethanol or water, which is a solution ingredient to dissolve or disperse the hair setting resin.

## Description

### TECHNICAL FIELD

The present invention relates to a solid or powder hairdressing composition. More specifically, it relates to a hairdressing that is used on towel-dried wet hair after hair washing, not a conventional liquid or paste hairdressing but a solid or powder hairdressing that contains none or drastically reduced amount of ethanol or water, which is a solution ingredient to dissolve or disperse the hair setting resin.

### BACKGROUND ART

A hairdressing is a hair cosmetic used to finalize the hair style and set the hair. That is, it is a cosmetic that is applied on dry hair for hair setting; a hairdressing is commonly liquid, gel, paste, or cream and examples include a hair liquid, which is a styling agent (for example, a hair setting agent resin) dissolved in a large quantity of ethanol and/or water, a gel-like hair gel, and a paste-like or cream-like hair wax.

For example, Patent Document 1 discloses a hair cosmetic containing spherical silicone and mono- or poly-glycerin or derivatives thereof, whose object is to improve the hair setting ability. Its claim 7 includes an example of a hair gel containing 20 wt% of ethanol and 65 wt% or more of water, which is a large quantity. Its claim 10 includes an example of a hair wax containing 65 wt% or more of water, which is a large quantity.

Patent Document 2 discloses a hairdressing agent composition containing (A) one, two, or more selected from waxes and fats and oils having a melting point of 20-50°C and (B) an alkyl-modified silicone. Its claim 12 includes an example of a hair wax containing 5 wt% of ethanol and 40 wt% or more of water, which is a large quantity. Its claim 13 includes an example of a hair gel containing 15 wt% of ethanol and 65 wt% or more of water, which is a large quantity.

Non-Patent Document 1 lists various hairdressings such as hair foam, hair mousse, hair spray, hair gel, set lotion, hair liquid, and hair wax as types of hair styling agents. These hairdressing compositions contain a large quantity of a solvent such as water or ethanol.

Conventionally, there is a solid hair wax called pomade or chick, which sometimes does not contain a large quantity of water or ethanol ingredients. This hairdressing is a mixture of solid wax and liquid oil components that assumes the form of paste to soft solid; but the main hairdressing ingredient is wax and it is fundamentally different from the styling agent (such as hair setting agent resin) used as the main hairdressing ingredient in the present invention.
In the present invention, the solid or powder hairdressing composition does not include a solid hair wax that uses solid wax, such as pomade and chick. In the present invention, the solid or powder hairdressing composition means a solid-like hairdressing composition that has, as the main ingredient, a styling agent (such as a hair setting agent resin) different from solid wax used for pomade and chick, to which no water or ethanol is added or a prescribed small amount of water and/or ethanol is added.

### {Prior art documents}

### {Patent Documents}

Patent Document 1: JP 2000-191452 A
Patent Document 2: JP 2007-169230 A {Non-Patent Documents}

Non-Patent Document 1: Editor: Takeo Mitsui, "Shin-Keshohin-Gaku [New Cosmetic Science] (second edition)", Nanzando, March 20, 2009, pp. 453-459.

### DISCLOSURE OF INVENTION

### TECHNICAL PROBLEM

As mentioned above, conventional hairdressings (hair styling products) range from solution-like to cream-like products and there are formulation forms ranging from low viscosity to high viscosity, but there is no hairdressing that contains a hair setting agent resin as a hairdressing ingredient and is completely solid.
Also, these styling products contain a large quantity of water and/or alcohol and therefore, when used on wet hair after hair washing, for example, drying is slow and the setting ability decreases.
The hairdressing composition of the present invention overturns the preconditions of conventional hairdressings, i.e. being liquid hairdressing mainly used for dry hair before setting (for example, used on the hair dried with a hair dryer after hair washing and towel drying); it provides a solid or powder hairdressing that fundamentally does not use much, if any, water and/or alcohol, which is directly used on wet hair to manifest an appropriate degree of setting ability.
Also, the hairdressing composition of the present invention has another advantage in that the container can be very small because a solvent such as water and/or alcohol is not used.

The present invention is, in contrast to the conventional concept of hairdressing, a solid or powder hairdressing composition; it does not contain, or contains a very small quantity of, water and/or alcohol and its object is to provide a hairdressing that mainly sets wet hair.
Another object is to provide a new hairdressing that can be used not only for wet hair but also for dry hair: for example, the hairdressing composition of the present invention can be mixed with water on the palm and then applied on the hair to achieve an appropriate degree of hairdressing.
Yet another object is to provide a hairdressing that allows fewer ingredients of the hairdressing and much more compact packaging by essentially not having water or ethanol, which are contained in conventional hairdressings in large quantities.

### TECHNICAL SOLUTION

That is, the present invention provides a solid or powder hairdressing composition comprising the following (a) ingredient and (b) ingredient:
(a) hair styling agent, and
(b) polyethylene glycol having a mass-average molecular weight of 1,000-25,000.

Also, the present invention provides the aforementioned hairdressing composition wherein the blend ratio of (c) water and/or ethanol is 5 wt% or less.

Furthermore, the present invention is a method of setting the hair using the aforementioned hairdressing composition wherein said hairdressing composition is applied as is on wet hair, not on dry hair, or applied on the hair after it is mixed with water on the palm.

### ADVANTAGEOUS EFFECTS

(1) The present invention is a hairdressing that manifests an appropriate degree of setting ability when used as is on moderately wet hair. For example, even after hair washing, it can be used directly on non-dry hair after towel drying to manifest a superior hairdressing ability.
(2) The present invention does not contain water or ethanol or their blend ratios are low, and therefore, when used on wet hair after hair washing, there is no problem of slow drying and a reduction in the setting ability.
(3) The present invention manifests a superior hairdressing ability on dry hair as well when the hairdressing composition of the present invention is mixed with water on the palm and then applied on the hair.
(4) The hairdressing composition of the present invention has an advantage in that the container can be very small because a solvent such as water and/or alcohol is not used.

### BEST MODE FOR CARRYING OUT THE INVENTION

### <(a) Hair styling agent>

In the present invention, the hair styling agent is the main ingredient for hairdressing (hair setting). The most preferable hair styling agent is a hair setting agent resin.
The solid or powder hairdressing composition of the present invention is a hairdressing that is fundamentally different from a solid hair wax, which has solid wax for the hairdressing ingredient; it stands for a hairdressing composition prepared by solidifying the hair setting agent resin preferably used in the (a) ingredient with the (b) ingredient, a solidifying agent, to obtain a solid hairdressing composition, or additionally crushing this to obtain a powder hairdressing composition.
Selection of the hair setting agent resin used in the present invention is not limited in particular; ingredients that are usually used in a hairdressing as a hair setting agent resin can be used. Examples of the hair setting agent resin include polyvinylpyrrolidone, polyvinylpyrrolidone/vinyl acetate copolymer, carboxyvinyl polymer, acrylic acid/alkyl methacrylate copolymer, octyl acrylamide/hydroxypropyl acrylate/butylaminoethyl methacrylate copolymer, vinylpyrrolidone/N,N-dimethylaminoethyl methacrylate/stearyl acrylate/tripropylene glycol diacrylate copolymer, 0-[2-hydroxy-3-(trimethylammonio)propyl]hydroxyethyl cellulose chloride, xanthan gum, hydroxyethyl cellulose, cross-linked N,N-dimethylacrylamide-2-acrylamide-2-methylpropanesulfonic acid sodium salt copolymer, N-methacryloyloxyethyl N,N-dimethylammonium-α-N-methylcarboxybetaine/methacrylic acid alkyl ester copolymer, polyvinylcaprolactam, polyoxyethylene/methylpolysiloxane copolymer, acrylic resin alcanolamine, and (polyurethane-24/methyl methacrylate) crosspolymer.
Preferably used commercial products include PVP K-30 (from ISP Japan Ltd.), PVP/VA S-630 (from Kaseihin Shoji Co. Ltd.), ACORN M deodorant (from Osaka Organic Chemical Industry Ltd.), Carbopol 980 (from Nikko Chemicals Co.), Carbopol 981 (from Nikko Chemicals Co.), Syntalen L (from 3V SIGMA), Syntalen K (from 3V SIGMA), Pemulen TR-1 (from B. F. Goodrich Chemical Company), Pemulen TR-2 (from B.F. Goodrich Chemical Company), CG Polymer (HV) (from Osaka Organic Chemical Industry Ltd.), Polymer JR-400 (from Union Carbide Japan), Katinal PC-100 (from Toho Chemical Industry Co., Ltd.), Keltorol (from Kelco), Keltorol T (from Kelco), Nomucoat ZZ (from Nisshin Oillio Ltd.), Natrosol 250 HHR (from Hercules Inc.), SU polymer G-1 (from Toho Chemical Industry Co., Ltd.), Yukaformer WPS (from Mitsubishi Chemical Corporation), Yukaformer R-205S (from Mitsubishi Chemical Corporation), Yukaformer 104-D (from Mitsubishi Chemical Corporation), RAM Resin-4000 (from Osaka Organic Chemical Industry Ltd.), Luviskol Plus (from BASF Japan Ltd.), Iodosol PUD (from Nippon NSC), Plus Size L-9909B (from Goo Chemical Co. Ltd.), and Aqualinker SU150A (from Konishi Co., Ltd.).

Preferable examples of hair styling agents in the present invention other than the hair setting agent resin include sugars or sugar alcohols such as erythritol, fructose, sorbitol, sucrose, maltitol, trehalose, glucose, and polyoxyethylene methyl glucoside, as well as activators such as polyoxyethylene polyoxypropylene decyltetradecyl ether, polyoxyethylene cetyl ether, propylene glycol laurate, polyoxyethylene (55) polyoxypropylene (28) dimethyl ether, polyoxyethylene hydrogenated castor oil, and polyoxypropylene decaglyceryl ether.
Preferably used commercial products include Glucam E-10J Humectant (from Ikeda Bussan Co., Ltd.), S-Safe 1324D (from NOF corporation), S-Safe 2010D (from NOF Corporation), EMALEX 125 (from Nihon Emulsion), Rikemal PL-100 (from Riken Vitamin), Macbiobride E-4060 (from NOF Corporation), EMALEX HC-60 (from Nihon Emulsion), and Beltamol DG-25 (from NOF Corporation).

### <Blend ratio>

In the present invention, the blend ratio of the hair styling agent is preferably 1-50 wt%, more preferably 5-40 wt%, most preferably 10-30 wt% relative to the total amount of the hairdressing composition.

### <(b) polyethylene glycol having a mass-average molecular weight of 1,000-25,000>

When the hairdressing composition is to be solid, polyethylene glycol having a mass-average molecular weight of 1,000-25,000 is used. When the solid is to be crushed into a powder form, polyethylene glycol having a mass-average molecular weight of 4,000-25,000 is preferable. If the mass molecular weight is too low, less than 4,000, then it may be difficult to process the solid into a powder form.
The mass-average molecular weight in the present invention means the following average molecular weight evaluated according to the Japanese Standards of Quasi-drug Ingredients.
Approximately 12.5 g of the sample is precisely weighed and put into an approximately 200 ml of pressure resistant ground-in stopper bottle, to which approximately 25 ml of pyridine is added and dissolved by heating, followed by cooling naturally. Separately, 42 g of phthalic anhydride is added to a 1 litter light resistant ground-in stopper bottle having 300 ml of pyridine, newly distilled and precisely weighed, dissolved by vigorous shaking, and let alone for 16 hours or more. Precisely weighed 25 ml of this liquid is added to the aforementioned ground-in stopper bottle, followed by hermetic sealing; the bottle is then enclosed with rugged cloth and put into a water bath that has been heated up to 98±2°C. When doing this, care is taken to make sure the liquid in the bottle is under the water bath surface. After maintaining the bottle for 30 minutes at 98±2°C, it is taken out of the water bath and let cool down to room temperature in the air. A precise quantity of 50 ml of a 0.5 mol/l sodium hydroxide solution is added, and the resulting solution is titrated with a 0.5 mol/l sodium hydroxide solution (indicator: a pyridine solution of phenolphthalein (1 -> 100) 5 drops). The end point of the titration is when the solution continuously assumes a light red color for 15 seconds. The blank test is conducted with the same method. Average molecular weight = (S*4000)/(a-b)
Where,
S: Amount of the sample (g)
a: Consumption of the 0.5 mol/l sodium hydroxide solution in the blank test (ml)
b: Consumption of the 0.5 mol/l sodium hydroxide solution in the sample test (ml)

Preferably used commercial products include polyethylene glycol 20000 (hereafter referred to as PEG-20000) (from Sanyo Chemical Industries Ltd.), polyethylene glycol 11000 (from NOF Corporation), and polyethylene glycol 6000 (from Sanyo Chemical Industries Ltd.), and polyethylene glycol 4000 (hereafter referred to as PEG-4000) (from Toho Chemical Industry Co., Ltd.).
In any case, commercially available polyethylene glycol PEG4000-PEG20000 can be used preferably for the hairdressing of the present invention; either it is to be solid or turned into a powder form by using a powderizing device (a mill or a file-like pulverizer).

### <Blend ratio>

In the present invention, the blend ratio of the aforementioned polyethylene glycol is preferably 40-90 wt%, more preferably 50-80 wt%, most preferably 50-70 wt% relative to the total amount of the hairdressing composition.

### <(c) Water and/or ethanol>

The blend ratio of water and/or ethanol must be 5 wt% relative to the total amount of the hairdressing composition if it is to be in a solid form. Whether water is blended in, ethanol is blended in, or water and ethanol are blended in with any ratio, it is an essential requirement of the present invention to keep the blend ratio at 5 wt% or less relative to the total amount of the hairdressing composition.
If the blend ratio is 5 wt% or less, then a hairdressing composition composed of the essential ingredients that are substantially the main ingredients, i.e. the aforementioned (a) ingredient and (b) ingredient can maintain a solid form. For powderizing the solid hairdressing composition, a hard solid is suitable (i. e. the blend ratio of water and/or ethanol is preferably low or zero); the blend ratio of water and/or ethanol is preferably 3 wt% or less relative to the total amount of the hairdressing composition.
In the present invention, it is also even more preferable to prepare a solid or powder hairdressing composition without substantially blending in water and/or ethanol.
As mentioned in the aforementioned "background art," a conventional hairdressing containing a setting agent resin contains a large quantity of water and/or ethanol and the blend ratio is far from 5 wt% or less, which is the blend ratio in the present invention.

### <(d) Oil component>

In the present invention, the addition of an appropriate amount of an oil component gives smoothness and gloss to the hairdressing and therefore embodiments that additionally use an oil component are also preferable.
Selection of the oil component is not limited in particular; preferable examples include liquid paraffin, cetyl 2-ethylhexanoate, squalane, isopropyl myristate, cetyl octanoate, octyl dodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, tri-2-heptyl undecanoic acid glyceride, castor oil fatty acid methyl ester, oleyl oleate, aceto glyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, chain polysiloxanes (for example, dimethylpolysiloxane, methylphenyl polysiloxane, and diphenyl polysiloxane), ring polysiloxanes (for example, octamethylcyclotetrasiloxane, decamethyl cyclopenta siloxane, and dodecamethyl cyclohexa siloxane), silicone resins forming a three-dimensional network structure, silicone rubbers, and various modified polysiloxanes (amino-modified polysiloxane, polyether-modified polysiloxane, alkyl-modified polysiloxane, and fluorine-modified polysiloxane). The blend ratio is determined appropriately and not limited in particular; it is preferably 5.0 wt% or less relative to the total amount of the hairdressing composition: 0.01-5.0 wt% is preferable.

In addition to the aforementioned essential ingredients, other ingredients used in cosmetics can be blended as necessary in the hairdressing composition of the present invention; examples of such ingredients include powder ingredients, humectants, thickeners, ultraviolet absorbents, sequestering agents, polyhydric alcohols, sugars, amino acids, organic amines, polymer emulsions, pH adjusting agents, skin nutrients, vitamins, antioxidants, antioxidation assistants, and perfumes, and the hairdressing composition can be prepared for the target formulation with a conventional method.
However, other than the aforementioned (a) ingredient for the purpose of setting the hair and the (b)-(c) ingredients for the purpose of solidifying it, the hairdressing composition of the present invention does not have to use ingredients for such purposes.
However, in the present invention, the addition of an appropriate amount of a thickener makes it possible to obtain a hairdressing that has a gel-like texture when applied on wet hair and therefore an embodiment in which a thickener is additionally used is also one of the preferable embodiments of the present invention. Selection of the thickener is not limited in particular; an acrylic thickener (for example, a copolymer of dimethylacrylamide and 2-acrylamide-2-methylpropanesulfonic acid) is preferable. The blend ratio is appropriately determined with no particular limitation; preferable is 5 wt% or less relative to the total amount of the hairdressing composition.

The present invention also provides a method for using the hairdressing composition wherein the aforementioned solid or powder hairdressing composition is used to set the hair.
Commonly, a hairdressing is a cosmetic that is used on dry hair to set the hair. However, the present invention is preferably applied on wet hair for hairdressing. It is particularly preferable to use it on wet hair after towel drying.
Or, for dry hair, one of the preferable embodiments of the present invention is to mix said hairdressing composition with water on the palm and apply this mixture with water on dry hair.
The method of using the hairdressing composition to set the hair according to the present invention provides an epoch making method that overturns the conventional commonsense; a person skilled in the art can never come up with this method.

### EXAMPLES

The present invention is described further in detail below by referring to Examples. The present invention is not limited to these Examples. The blend ratios in Examples are in wt% (mass-percentage) units unless specified otherwise.
Formulations in Table 1 were used in a conventional method to prepare solid or powder hairdressing compositions; solidification (powderization) in relation to the blend ratios of water and/or ethanol to turn them into a solid or powder form and the hair setting ability of the hairdressing composition of the present invention were investigated. First, the evaluation methods are described below.

### <Methods to evaluate solidification or powderization>

In the present invention, "solid" means a degree of hardness where the shape does not change when lightly pushed by a finger (hardness comparable to common solid soap). For the powder, a pepper mill (apparatus) was used to process the solid composition into powder.
"Evaluation"
○: It is possible to process it to be solid or powder.
○Δ, Δ: It is possible to process it to be solid. It is possible to process it to be soft powder.
×: It is not possible at all to process it to be solid.

### <Evaluation of ease of application on wet hair and hair setting ability>

An actual use test was conducted with (two) panelists: the sample was applied on wet hair after hair washing and light towel-drying and ease of application on the hair and the hair setting ability were evaluated.
"Evaluation of ease of application on wet hair"
○: Panelists (both of the two) felt that application and absorption on wet hair after towel-drying were very easy.
Δ: Panelists (both of the two) felt that application and absorption on wet hair after towel-drying were easy.
×: Panelists (both of the two) felt that application and absorption on wet hair after towel-drying were not easy.
"Evaluation of hair setting ability on wet hair"
○: Panelists (both of the two) could easily accomplish a desired hair setting.
Δ: Panelists (both of the two) had a hard time accomplishing a desired hair setting.
×: Panelists (both of the two) could not accomplish a desired hair setting at all.

**[Table 1]**

| | Example 1 | Example 2 | Example 3 | Example 4 | Example 5 | Comparative example 1 |
|---|---|---|---|---|---|---|
| (a) Setting agent resin PVP/VA *1 | 20 | 19.9 | 19.8 | 19.4 | 19 | 18 |
| (b) PEG-4000 | 80 | 79.6 | 79.2 | 77.6 | 76 | 72 |
| (c) Water | 0 | 0.5 | 1 | 3 | 5 | 10 |
| Solidification or powderization | ○ | ○ | ○ | ○ | Δ | × |
| Ease of application on wet hair | ○ | ○ | ○ | ○ | Δ | × |
| Hair setting ability on wet hair | ○ | ○ | ○ | ○ | ○ | ○ |

| | | | | | | |
|---|---|---|---|---|---|---|
| *1: Polyvinylpyrrolidone/vinyl acetate copolymer PVP/VA S-630 (from Kaseihin Shoji Co. Ltd.) | | | | | | |

**[Table 2]**

| | Example 1 | Example 6 | Example 7 | Example 8 | Example 9 |
|---|---|---|---|---|---|
| (a) Setting agent resin PVP/VA *1 | 20 | 19.9 | 19.8 | 19.4 | 19 |
| (b) PEG-4000 | 80 | 79.6 | 79.2 | 77.6 | 76 |
| (d) Ethanol | 0 | 0. 5 | 1 | 3 | 5 |
| Solidification or powderization | ○ | ○ | ○ | ○Δ | Δ |
| Ease of application on wet hair | ○ | ○ | ○ | ○ | Δ |
| Hair setting ability on wet hair | ○ | ○ | ○ | ○ | ○ |

| | | | | | |
|---|---|---|---|---|---|
| *1: Polyvinylpyrrolidone/vinyl acetate copolymer PVP/VA S-630 (from Kaseihin Shoji Co. Ltd.) | | | | | |

**[Table 3]**

| | Example 1 | Example 10 | Example 11 | Example 12 |
|---|---|---|---|---|
| (a) Setting agent resin PVP/VA *1 | 20 | 19.9 | 19.8 | 19.4 |
| (b) PEG 4000 | 80 | 79.6 | 79.2 | 77.6 |
| (e) Liquid paraffin | 0 | 0.5 | 1 | 3 |
| Solidification or powerization | ○ | ○ | ○ | ○ |
| Ease of application on wet hair | ○ | ○ | ○ | ○ |
| Hair setting ability on wet hair | ○ | ○ | ○ | ○ |

| | | | | |
|---|---|---|---|---|
| *1: Polyvinylpyrrolidone/vinyl acetate copolymer PVP/VA S-630 (from Kaseihin Shoji Co. Ltd.) | | | | |

**[Table 4]**

| | Example 1 | Example 13 | Example 14 | Example 15 |
|---|---|---|---|---|
| (a) Setting agent resin PVP/VA *1 | 20 | 19.9 | 19.8 | 19.4 |
| (b) PEG-4000 | 80 | 79.6 | 79.2 | 77.6 |
| (f) Cetyl 2-ethylhexanoate | 0 | 0.5 | 1 | 3 |
| Solidification or powderization | ○ | ○ | ○ | ○ |
| Ease of application on wet hair | ○ | ○ | ○ | ○ |
| Hair setting ability on wet hair | ○ | ○ | ○ | ○ |

| | | | | |
|---|---|---|---|---|
| *1: Polyvinylpyrrolidone/vinyl acetate copolymer PVP/VA S-630 (from Kaseihin Shoji Co. Ltd.) | | | | |

**[Table 5]**

| | Example 1 | Example 16 | Example 17 | Example 18 |
|---|---|---|---|---|
| (a) Setting agent resin PVP/VA *1 | 20 | 19.9 | 19.8 | 19.4 |
| (b) PEG-4000 | 80 | 79.6 | 79.2 | 77.6 |
| (g) Silicone KF-96A6T | 0 | 0.5 | 1 | 3 |
| Solidification or powderization | ○ | ○ | ○ | ○ |
| Ease of application on wet hair | ○ | ○ | ○ | ○ |
| Hair setting ability on wet hair | ○ | ○ | ○ | ○ |

| | | | | |
|---|---|---|---|---|
| *1: Polyvinylpyrrolidone/vinyl acetate copolymer PVP/VA S-630 (from Kaseihin Shoji Co. Ltd.) | | | | |

**[Table 6]**

| | Example 1 | Example 19 | Example 20 | Example 21 |
|---|---|---|---|---|
| (a) Setting agent resin PVP/VA *1 | 20 | 19.9 | 19.8 | 19.4 |
| (b) PEG-4000 | 80 | 79.6 | 79.2 | 77.6 |
| (h) S-Safe 2010D *2 | 0 | 0.5 | 1 | 3 |
| Solidification or powderization | ○ | ○ | ○ | ○ |
| Ease of application on wet hair | ○ | ○ | ○ | ○ |
| Hair setting ability on wet hair | ○ | ○ | ○ | ○ |

| | | | | |
|---|---|---|---|---|
| *1: Polyvinylpyrrolidone/vinyl acetate copolymer PVP/VA S-630 (from Kaseihin Shoji Co. Ltd.) *2: Polyoxyethylene polyoxypropylene decyltetradecyl ether S-Safe 2010D (from NOF corporation) | | | | |

Other Examples of the solid or powder hairdressing composition of the present invention are shown below. All Examples are hairdressings that have a superior hairdressing ability on moderately wet hair after hair washing and towel drying.

### [Example 22: Solid or powder hairdressing]

| Ingredient | wt% |
|---|---|
| Polyvinylpyrrolidone/vinyl acetate copolymer (PVP/VA-S630) | 24 |
| Cross-linked N,N-dimethylacrylamide-2-acrylamide- | |
| 2-methylpropanesulfonic acid sodium salt copolymer^{*1} | 13 |
| PEG4000 | 60 |
| Menthol | 1 |
| Propylene glycol | 1 |
| Perfume | 1 |

### Preparation method:

PEG4000 is dissolved at 60-70°C. Other raw materials were added and stirred to be homogeneously dispersed, followed by solidifying by rapid cooling, to obtain a solid form. The solid composition can be pulverized with a powder mill to obtain a powder form.
*1: 35 g of dialkylacrylamide (from Kohjin), 17.5 g of 2-acrylamide-2-methylpropanesulfonic acid (from Sigma), and 70 mg of methylene bisacrylamide are dissolved in 260 g of ion-exchanged water and the pH is adjusted to 7.0 with sodium hydroxide. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) are put into a 1,000 ml three-neck flask provided with a refluxing apparatus, mixed and dissolved, and then subjected to N₂ substitution. The monomer aqueous solution is added to this three-neck flask, and the temperature is raised to 65°C-70°C using an oil bath as stirring is carried out in an N₂ atmosphere. When the system temperature reaches 65°C-70°C, after confirming that the system has taken on a semitransparent microemulsion state, 2 g of ammonium persulfate is added to the polymerization system to start the polymerization. The temperature of the polymerization system is maintained at 65°C-70°C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under reduced pressure to obtain the dried microgel in a white powder form.

### [Example 23: Solid or powder hairdressing]

| Ingredient | wt% |
|---|---|
| Polyvinylpyrrolidone/vinyl acetate copolymer (PVP/VA-S630) | 25 |
| cross-linked N,N-dimethylacrylamide-2-acrylamide- | |
| 2-methylpropanesulfonic acid sodium salt copolymer^{*1} | 10 |
| PEG4000 | 60 |
| Water | 5 |

### Preparation method:

PEG4000 is dissolved at 60-70°C. Other raw materials were added and stirred to be homogeneously dispersed, followed by solidifying by rapid cooling, to obtain a solid form. The solid composition can be pulverized with a powder mill to obtain a powder form.
*1: Same as Example 22.

### INDUSTRIAL APPLICABILITY

The present invention can provide a hairdressing that manifests an appropriate degree of setting ability when used on wet hair. For example, even after hair washing, it can be used directly on non-dry hair after towel drying to manifest a superior hairdressing ability.
Also, the present invention manifests a superior hairdressing ability on dry hair as well when the hairdressing composition of the present invention is mixed with water on the palm and then applied on the hair.
Furthermore, the hairdressing composition of the present invention has an advantage in that the container can be very small because a solvent such as water and/or alcohol is not used.
Based on the description above, the new hairdressing of the present invention has a very high possibility of industrial applications.

## Claims

1. A solid or powder hairdressing composition comprising the following (a) ingredient and (b) ingredient:
(a) hair styling agent, and
(b) polyethylene glycol having a mass-average molecular weight of 1,000-25,000.

2. The hairdressing composition of claim 1
wherein the blend ratio of (c) water and/or ethanol is 5 wt% or less.

3. A method of setting the hair using the hairdressing composition of claim 1 or 2 wherein said hairdressing composition is applied as is on wet hair, not on dry hair, or applied on the hair after it is mixed with water on the palm.
